# EUROPEAN PATENT APPLICATION

(11) **EP 2 444 105 A1**
(43) Date of publication of application: **25.04.2012**
(21) Application number: 10789519.5
(22) Date of filing: 16.06.2010
(51) Int. Cl.: A61K 45/00, A61K 38/00, A61K 45/06, A61P 31/04

(54) **ANTI-GRAM-NEGATIVE BACTERIA AGENT**

(30) Priority: 16.06.2009 JP 2009143600
(71) Applicant: Tokai University, Tokyo 151-0063 (JP)
(72) Inventor: YOSHIHARA, Eisaku, Hiratsuka-shi Kanagawa 259-1212 (JP); MITSUNAGA, Shigeki, Yokohama-shi Kanagawa 227-0036 (JP); INOKO, Hidetoshi, Yokohama-shi Kanagawa 245-0002 (JP)
(74) Representative: Docherty, Robert Charles
(86) International application number: PCT/JP2010/060207
(87) International publication number: WO 2010/147145

(57) **Abstract**

Provided are: a method for blocking the biosynthesis of an outer membrane protein (OMP) necessary for the survival of Gram-negative bacteria by inhibiting the formation of a YaeT complex in the outer membrane of the bacteria and an agent therefor for the purpose of basically solving a problem of the development of multidrug resistance in Gram-negative bacteria. Specifically disclosed is an anti-Gram-negative bacteria agent, wherein the agent exerts a bactericidal action, a growth-inhibiting action, and/or a drug efflux-inhibiting action on Gram-negative bacteria by inhibiting the formation of a YaeT complex. The agent is preferably a peptide molecule comprising an amino acid sequence consisting of at least LTLR or a peptide molecule comprising an amino acid sequence consisting of at least FIRL.

## Description

### Technical Field

The present invention relates to an effective agent for preventing/treating Gram-negative bacterial infection. More particularly, it relates to an agent capable of exerting a bactericidal action, a growth-inhibiting action, and/or a drug efflux-inhibiting action on Gram-negative bacteria by inhibiting the formation of a YaeT complex present in the outer membrane of the Gram-negative bacteria.

### Background Art

Multidrug resistant Gram-negative bacteria such as Pseudomonas aeruginosa and Acinetobacter baumannii are the major causative bacteria of nosocomial infectious diseases and sometimes lead to serious pathologic conditions in patients having reduced immune strength. Because of their multidrug resistance, infections with these bacteria are difficult to treat, and they have been a major problem. Gram-negative bacteria have two membranes, the inner membrane and the outer membrane. Therefore, for effective action, an antibiotic is required to pass through the complex membrane structure of the Gram-negative bacteria and reach the interior of the cells. However, it is considered that a drug-modifying enzyme, a reduction in the permeability of the outer membrane, an alteration in the target, drug efflux by a drug efflux pump, and the like lead to a reduction in the effect of the antibiotic, and thereby the acquisition of the multidrug resistance.

A multidrug efflux pump greatly contributes to the acquisition of the multidrug resistance by Pseudomonas aeruginosa. This pump, using energy, actively transports and discharges drugs entered into the bacteria to the exterior of the cells. The multidrug efflux pump is divided into several families; among these, a pump belonging to the RND (resistance nodulation division) family known to discharge various antibiotics consists of 3 types of subunits. A plurality of RND-type pumps is present in Pseudomonas aeruginosa; among these, the major pump is a MexAB-OprM pump.

The present inventors focused attention to the MexAB-OprM pump of Pseudomonas aeruginosa, developed a method for inhibiting the drug efflux pump function of Pseudomonas aeruginosa to enhance the efficacy of an antibiotic by modifying the amino acid sequence of OprM, a subunit constituting the multidrug efflux pump, and proposed an agent exerting such effect and a method for selecting such agent through screening (Patent Document 1). However, the occurrence of mutation in the target sequence has had the possibility of leading to the loss of the effect of the method.

Meanwhile, the research of Gram-negative bacteria including Pseudomonas aeruginosa and Escherichia coli has been progressing; as a result, the YaeT complex has been known to be essential for the biosynthesis of a β-barrel protein (a protein forming pores of the outer membrane) (Non-Patent Document 1) and the three-dimensional structure of the YaeT complex has also been elucidated (Non-Patent Document 2). YaeT is responsible for the biosynthesis of an outer membrane protein (OMP) by forming a complex with 4 lipoproteins, YfgL, YfiO, NlpB and SmpA. In other words, the inhibition of the complex formation of YaeT can probably block the transport of OMP including the drug efflux pump to the outer membrane.

### Citation List

### Patent Document

Patent Document 1: International Publication No. WO 2007/091395

### Non-Patent Document

Non-Patent Document 1: Seokhee Kim et al., Science, Vol. 317, pp.961-964 (2007)
Non-Patent Document 2: Rajeev Misra, ACS Chemical Biology, Vol. 2, pp.649-651 (2007)

### Summary of Invention

### Technical Problem

Made in view of the above-described circumstances, the present invention has an object of providing a method for blocking the transport of an outer membrane protein (OMP) necessary for the survival of Gram-negative bacteria by inhibiting the formation of a YaeT complex in the outer membrane of the bacteria and an agent therefor to basically solve a problem of the development of multidrug resistance in Gram-negative bacteria.

### Solution to Problem

Thus, the present invention provides an anti-Gram-negative bacteria agent, wherein the agent exerts a bactericidal action, a growth-inhibiting action, and/or a drug efflux-inhibiting action on Gram-negative bacteria by inhibiting the formation of the YaeT complex.

### Advantageous Effect of Invention

According to the present invention, a problem of the development of multidrug resistance due to the mutation of an outer membrane protein is basically solved because the YaeT complex formation in Gram-negative bacteria is inhibited in the presence and/or absence of a conventional antibiotic and thereby a bactericidal action, a growth-inhibiting action, and/or a drug efflux-inhibiting action are exerted on Gram-negative bacteria.

### Brief Description of Drawings

[Figure 1] Figure 1 is a schematic diagram describing the outer membrane and the inner membrane of Gram-negative bacteria and the relationship between a YaeT complex and an outer membrane protein (cited from Non-Patent Document 1).
[Figure 2] Figure 2 is a drawing showing a comparison of amino acid sequences of the sites of binding of YfgL to YaeT in various Gram-negative bacteria.
[Figure 3] Figure 3 is a drawing showing a comparison of amino acid sequences of the conservative regions of YfiO of various Gram-negative bacteria.
[Figure 4] Figure 4 is a graph showing the concentration dependency of the bactericidal action of LTLR and LTLR-NH₂ on strain PAO1.
[Figure 5] Figure 5 is a graph showing changes in the susceptibility of strain nalB to OFLX in the presence and absence of LTLR-NH₂ (5 mM).
[Figure 6] Figure 6 is a graph showing changes in the susceptibility of strain nalB to AZT in the presence and absence of LTLR-NH₂ (5 mM).
[Figure 7] Figure 7 is a graph showing changes in the susceptibility of strain nalB to OFLX in the presence and absence of FIRL-NH₂ (1.5 mM).
[Figure 8] Figure 8 is a graph showing changes in the number of bacterial cells in a corneal ulcer model (Example 7).
[Figure 9] Figure 9 is a photograph showing the appearance of corneas after 12 hours in a corneal ulcer model (Example 7).
[Figure 10] Figure 10 is a photograph showing the appearance of corneas after 24 hours in a corneal ulcer model (Example 7).
[Figure 11] Figure 11 is a graph showing changes in the clinical score in a corneal ulcer model (Example 7).
[Figure 12] Figure 12 is a graph showing changes in the number of bacterial cells in a pneumonia model (Example 8) .
[Figure 13] Figure 13 is a graph showing changes in the number of bacterial cells in a pneumonia model (Example 8) .

### Description of Embodiments

As described above, the anti-Gram-negative bacteria agent of the present invention is characterized by exerting a bactericidal action, a growth-inhibiting action, and/or a drug efflux-inhibiting action on Gram-negative bacteria by inhibiting the formation of the YaeT complex.

For the purpose of the present invention, the term "Gram-negative bacteria" is used in the commonly used sense. Thus, it is a generic term applied to bacteria whose crystal violet stain is decolorized in Gram staining. Typical Gram-negative bacteria include proteobacteria such as Escherichia coli, Salmonella, Pseudomonas, and Helicobacter, and cyanobacteria. When classified in connection with medicine, they include bacilli such as Pseudomonas aeruginosa and Hemophilus influenzae causing the disturbance of the respiratory system, Escherichia coli and Proteus mirabilis causing the disturbance of the urinary system, and Helicobacter pylori and Bacillus Gaertner causing the disturbance of the alimentary system and micrococci such as Neisseria meningitidis, Moraxella catarrhalis, and Neisseria gonorrhoea.

The YaeT complex in Gram-negative bacteria is formed by the assembly of the four lipoproteins YfgL, YfiO, NlpB and SmpA in the membrane protein YaeT. Particularly, for the biosynthesis of OMP by the YaeT complex, it is considered to be essential for YfgL and YfiO to bind thereto; the inhibition of the binding of them to YaeT will not result in the biosynthesis of OMP.

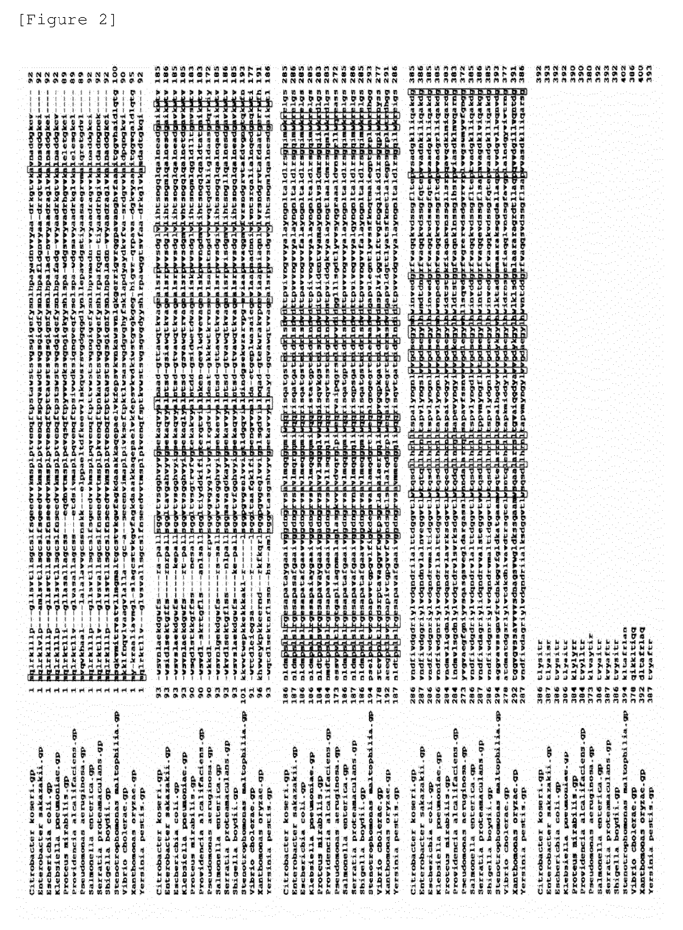

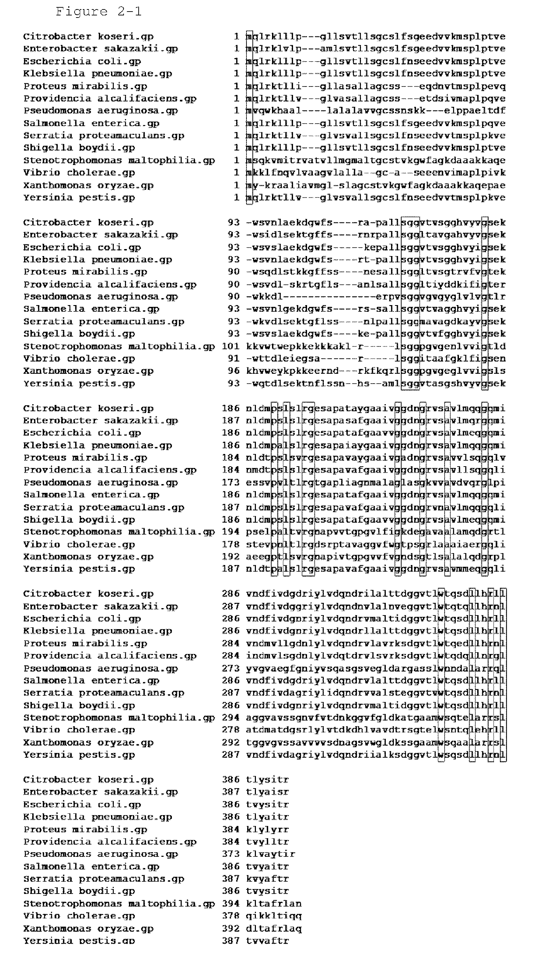

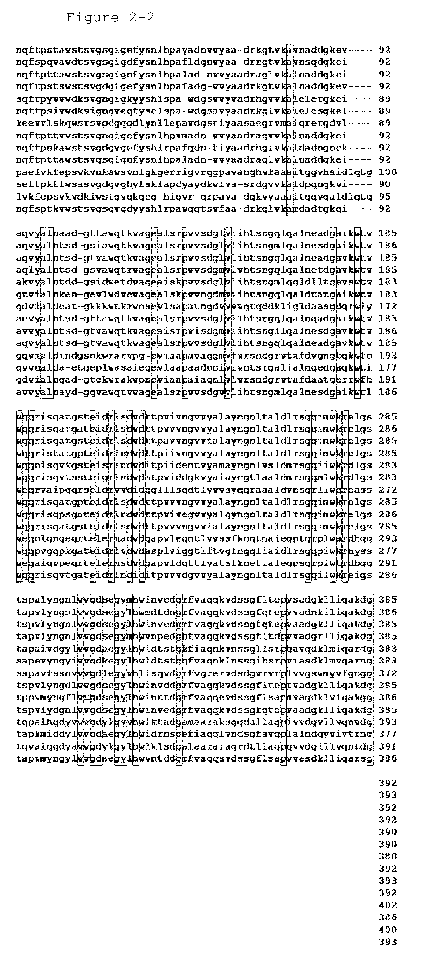

Thus, a first aspect of the present invention is an anti-Gram-negative bacteria agent, wherein the agent inhibits the binding of YaeT to YfgL.
In searching for an agent of this aspect, the present inventors compared the amino acid sequence of the sites of binding of YfgL to YaeT in various Gram-negative bacteria (see Figure 2) to examine the highly conserved regions thereof. In Figure 2, the boxed regions indicate highly conserved amino acid residues; various peptides have been synthesized based on the amino acid sequence of the region shown in "A" among these regions to determine the action thereof.

As a result, when a peptide molecule comprising the amino acid sequence "LTLR" is used, it has been found to be capable of competing with the binding of YaeT to YfgL to effectively inhibit the biding thereof. Thus, the anti-Gram-negative bacteria agent of the present invention is preferably a peptide comprising an amino acid sequence consisting of at least LTLR.

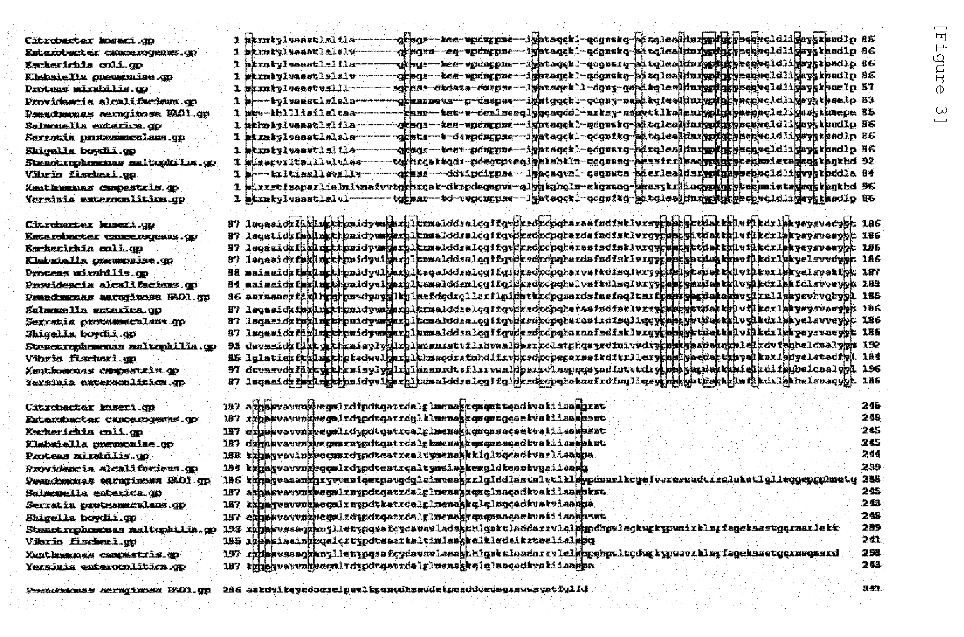

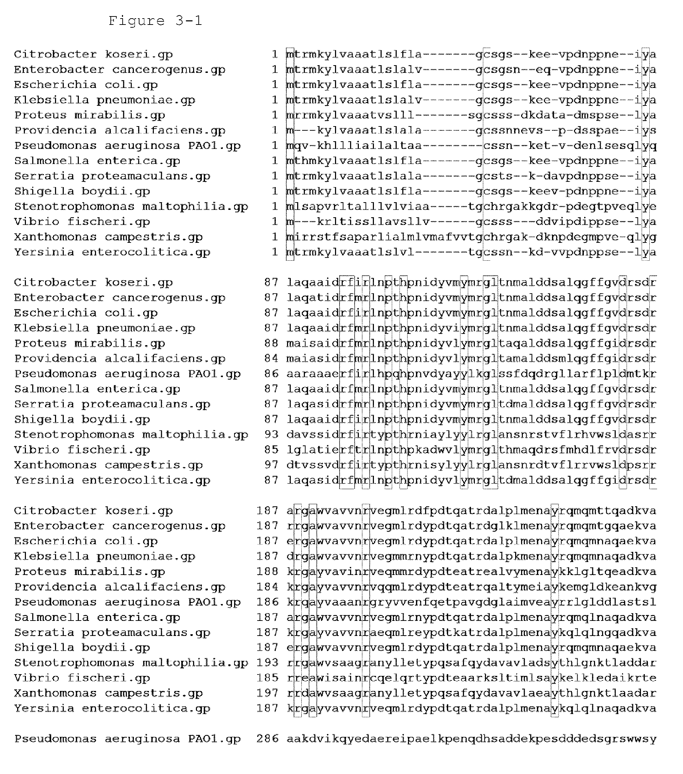

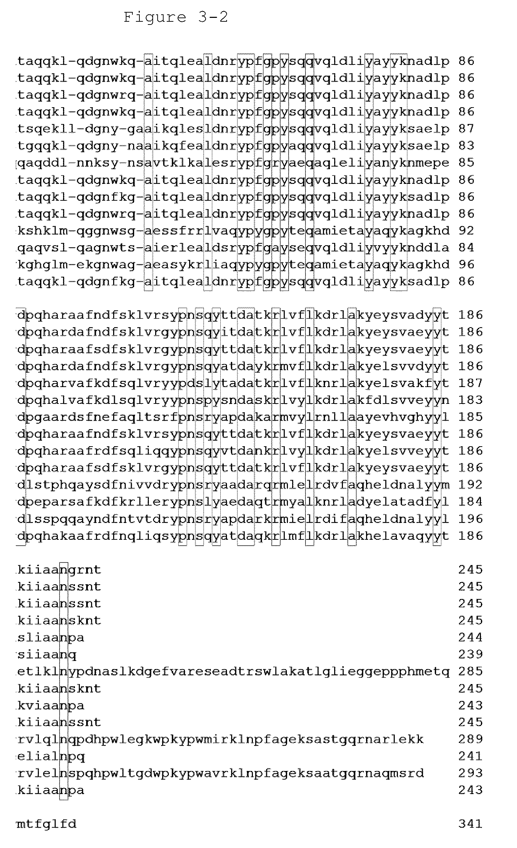

As a second aspect of the present invention, attention has then been directed to the inhibition of the binding of YaeT to YfiO. Figure 3 is a diagram showing a comparison of amino acid sequences of YfiO of various Gram-negative bacteria. A peptide molecule having an amino acid sequence, "FIRLHP", among the highly conserved regions has been found to be capable of competing with the binding YaeT to YfiO to effectively inhibit the binding thereof. It has also been determined that the peptide molecule is effective without comprising all of the amino acid sequence "FIRLHP" provided that it comprises a portion of the amino acid sequence, "FIRL" or "IRLH". Thus, the anti-Gram-negative bacteria agent of the present invention is preferably a peptide comprising an amino acid sequence consisting of at least FIRL or IRLH, particularly preferably a peptide comprising an amino acid sequence consisting of at least FIRLHP.

The peptide molecule may also be chemically modified. For example, the peptide molecule whose C-terminal is amidated has a dramatically improved bactericidal action compared to the unmodified peptide molecule. The present inventors believe that the improvement may result from the membrane permeability being enhanced by the cancellation of the C-terminal negative charge by the amido group.

On the contrary, however, the peptide molecule whose N-terminal is acetylated has a reduced bactericidal action.
Thus, the anti-Gram-negative bacteria agent of the present invention is preferably the peptide molecule whose C-terminal is amidated.

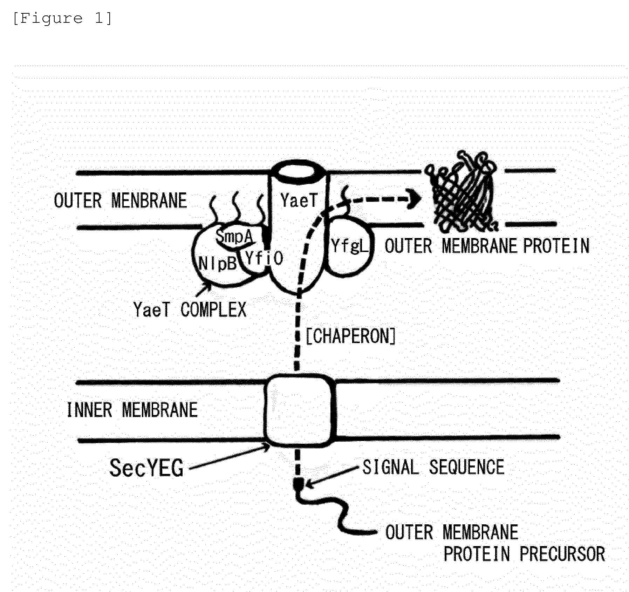

The site of the interaction between YaeT and each of YfgL and YfiO targeted in the present invention occur in the periplasm located between the outer membrane and the inner membrane of bacteria (see Figure 1). Thus, the agent (peptide molecule) of the present invention is required to pass through the outer membrane in order to reach the action site from the outside.
Particularly, the unmodified peptide molecule is preferably administered simultaneously with an agent enhancing the permeability of the outer membrane in order to deliver the efficacy at a practical concentration.

The agent enhancing the permeability of the outer membrane is not particularly limited; however, examples thereof include antibiotics having a cell membrane-altering action. Specific examples thereof include polymyxin B and colistin, etc.

In contrast, the peptide molecule whose C-terminal is amidated can probably pass through the outer membrane in itself since the negative charge of the C-terminal has disappeared as described above. In fact, the survival rate of bacteria has been demonstrated to be reduced even when aztreonam (a β-lactam agent), an antibiotic having no membrane-altering action, rather than polymyxin B has been simultaneously administered.

That is, when the amidated peptide molecule of the present invention is used, the peptide molecule probably passes through the outer membrane without simultaneous administration of the antibiotic having a membrane-altering action and reaches the site of formation of the YaeT complex to inhibit the biosynthesis of the outer membrane protein. Thus, for example, when an antibiotic having no membrane-altering action is simultaneously administered, the amidated peptide molecule of the present invention acts to weaken the membrane, probably resulting in increased susceptibility to the antibiotic and the exertion of a bactericidal effect.

Thus, a third aspect of the present invention is a therapeutic agent for Gram-negative bacterial infection, wherein the agent comprises an anti-Gram-negative bacteria agent (modified/unmodified) and an antibiotic (having a membrane-altering action or not).

The antibiotic having no membrane-altering action is not particularly limited and may use one conventionally in common use. Examples thereof include cephalosporin antibiotics such as cephalexin and cefotaxime, fluoroquinolone antibiotics such as ofloxacin and ciprofloxacin, aminoglycoside antibiotics such as amikacin, and carbapenem antibiotics such as meropenem.

### Examples

The present invention will be described below in further detail with reference to specific examples. However, the present invention is not intended to be limited to these Examples.
In Examples and the like described below, the number of viable cells of Gram-negative bacteria (Pseudomonas aeruginosa or Escherichia coli) is measured as follows.
A portion of bacteria cultured at 37°C overnight is added to a fresh MH medium and continues to be cultured at the same temperature. When the turbidity (absorbance at 600 nm) of the bacteria reaches 0.7 - 0.8, the bacterial solution is added an MH medium to prepare a bacterial solution diluted 1:100 - 200, which is used for experimental purposes.
A solution in which 60 - 80 µL of the bacterial solution was added to a solution of a peptide and the like to a final amount of 100 µL is cultured for a certain time (3 hours) while stirring at 37°C. After culture, the bacterial solution was diluted with a PBS solution, and the diluted solution is seeded on an MH agar plate and cultured at 37°C overnight. The following day, the number of colonies grown on the plate is counted to determine the number of viable bacterial cells.

### (Example 1)

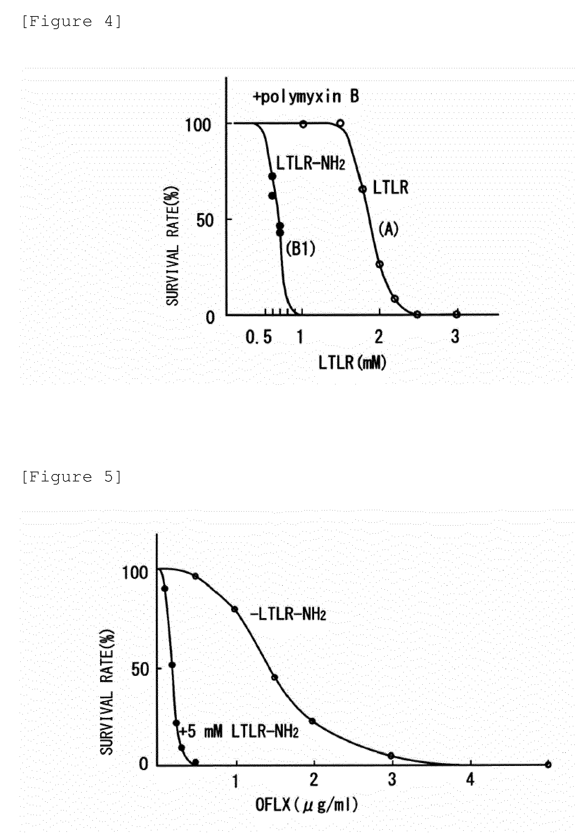

In the above experimental system, Pseudomonas aeruginosa (strain PAO1) was cultured after adding polymyxin B (final concentration: 0.4 µg/mL) (this concentration does not affect the survival of Pseudomonas aeruginosa). At this time, the culture was performed under conditions in which various concentrations of a peptide, LTLR (a peptide consisting of 4 amino acid residues, having the sequence leucine-threonine-leucine-arginine) were added. Under each condition, the number of viable cells of Pseudomonas aeruginosa was measured, and it was measured how the addition of the peptide changes the number of the viable cells compared to the number of the viable cells for no addition of the peptide. The results are shown in the graph of Figure 4 (A).

The survival rate of Pseudomonas aeruginosa was decreased in a manner dependent on the concentration of the peptide LTLR in the presence of 0.4 µg/mL polymyxin B; the survival rate of Pseudomonas aeruginosa was almost 0% when 2.5 mM LTLR was added for culture.

### (Comparative Example 1A)

The peptide LTLE, which results from substituting 4th amino acid (R) of the peptide, LTLR in Example 1 with glutamic acid (E), was synthesized, and a change in the number of viable bacterial cells under the addition of 2.5 mM LTLE was measured under the same conditions as those in Example 1. As a result, no decrease in the survival rate of Pseudomonas aeruginosa was observed. Thus, the antibacterial action observed in Example 1 was suggested to be specific for the amino acid sequence (LTLR).

### (Comparative Example 1B)

The peptide LRTL, which results from randomizing the amino acid sequence of the peptide LTLR, was synthesized in Example 1, and a change in the number of viable bacterial cells under the addition of 2.5 mM LRTL was measured under the same conditions as those in Example 1. As a result, no decrease in the survival rate of Pseudomonas aeruginosa was observed. Thus, the antibacterial action observed in Example 1 was suggested to be specific for the amino acid sequence (LTLR).

### (Example 2)

Clinical isolates (strains 24, 42, 83, 88 and 92 of P. aeruginosa), strain nalB (a strain having acquired high multidrug resistance by highly expressing MexAB-OprM as a drug efflux pump), and strain nfxC (a strain having acquired high multidrug resistance by highly expressing MexEF-OprN as a drug efflux pump) were used in place of the strain PA01 (standard strain) of Pseudomonas aeruginosa used in Example 1 to measure the action of LTLR thereon. In this experiment, the final concentration of polymyxin B was set at 0.2 µg/mL with 3 mM LTLR added to examine changes in the survival rate thereof. The results are shown in Table 1.

**[Table 1]**

| Strain | Survival Rate (%) |
|---|---|
| P.aeruginosa 24 | 0 |
| P.aeruginosa 42 | 43 |
| P.aeruginosa 83 | 0 |
| P.aeruginosa 88 | 25 |
| P.aeruginosa 92 | 9 |
| nalB | 0 |
| nfxC | 1 |

Even the clinical isolate, strain 42 of Pseudomonas aeruginosa, which exhibited a highest survival rate, had a survival rate of less than 50%, and even strains nalB and nfxC as "highly resistant strains" had a survival rate of almost zero, demonstrating that the peptide of the present invention was effective even on multidrug resistant bacteria.

### (Example 3)

Escherichia coli (ATCC25922) was used in place of Pseudomonas aeruginosa for the same measurement as in Example 2. As a result, the survival rate of Escherichia coli was found to be about 1%. This suggested that LTLR also acts on the whole Gram-negative bacteria including Escherichia coli as well as Pseudomonas aeruginosa.

### (Comparative Example 2)

LTLR whose amino terminal was acetylated was synthesized to examine the action thereof. The survival rate of Pseudomonas aeruginosa was examined under the same conditions as those in Example 1 by changing LTLR to the acetylate LTLR. As a result, no decrease in the survival rate thereof was observed, showing that the acetylation markedly reduced the anti-Gram negative activity.

### (Example 4)

LTLR whose carboxy terminal was amidated was synthesized to examine the action thereof. Under the same conditions as those in Example 1, various concentrations of the amidated LTLR were added to examine changes in the survival rate of Pseudomonas aeruginosa. The results are shown in Figure 4 (B). The results show that the amidation increased the activity of LTLR, demonstrating that the amidation of the carboxy terminal was also involved in an increase in the activity thereof as well as having a peptide-protecting action.
Similar experimental results were also obtained with Escherichia coli.

### (Comparative Example 3)

To examine how the changed length of LTLR affects activity, carboxy terminal-amidated TLR and LR were synthesized to measure the activity thereof. Under the same conditions as those in Example 4, the amidated TLR or LR was added at a concentration of 10 mM to examine the action thereof on Pseudomonas aeruginosa. As a result, no decrease in the bacterial survival rate was observed.

### (Example 5)

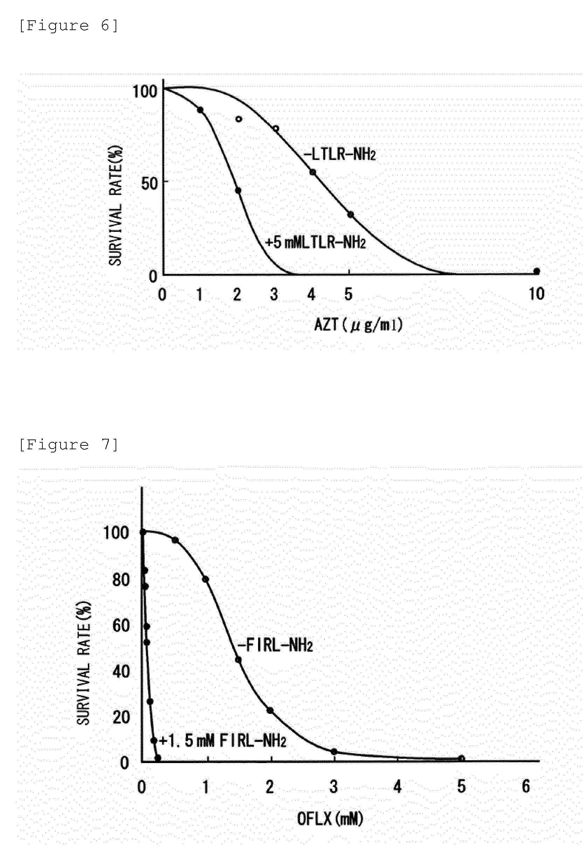

The same experiment as in Example 4 was carried out in the system using ofloxacin (OFLX) or aztreonam (AZT) as an antibiotic in place of polymyxin B having a membrane-altering action. The results are shown in Figures 5 and 6. In the system using either OFLX or AZT, the addition of the amidated peptide molecule (LTLR) dramatically decreased the survival rate of a multidrug resistant strain (NalB) of Pseudomonas aeruginosa at the same antibiotic concentration compared to that with no addition thereof. That is, it was suggested that the amidated peptide molecule of the present invention passed through the outer membrane, reached the target site, and weakened the bacteria. Thus, the amidated peptide molecule of the present invention was demonstrated to have an action enhancing the effect of an antibiotic such as aztreonam.

### (Example 6)

FIRL (Yf2B1), which inhibits the binding of YaeT to YfiO, was used as a peptide molecule to perform the same experiment with Pseudomonas aeruginosa (nalB) as in Example 1. The results are shown in Figure 7.
The peptide molecule inhibiting the binding of YaeT to YfiO was demonstrated to have a more excellent anti-Gram negative bacteria action.

The following animal experiments were carried out using the peptide FIRL and its amidated product as exemplary anti-Gram negative bacteria agents of the present invention.

### (Example 7)

### Experiment Using Rabbit Corneal Ulcer Model by Pseudomonas aeruginosa Infection

The center of the cornea of Japanese white rabbits (2 kg, male) was scratched and inoculated with multidrug resistant Pseudomonas aeruginosa (MDRP) to form ulcer. These rabbits were divided into the following 3 groups (of 5 rabbits each), and ocular instillation of the following agents was started in the respective groups after 6 hours of inoculation. The ocular instillation was carried out three times: 6, 12 and 18 hours after the inoculation with MDRP.
Group 1: saline (control)
Group 2: ofloxacin (OFLX) eye drops (Tarivid Otic Solution 0.3%, Santen Pharmaceutical Co., Ltd.)
Group 3: a mixed solution of OFLX eye drops and 50 mM FIRL/saline

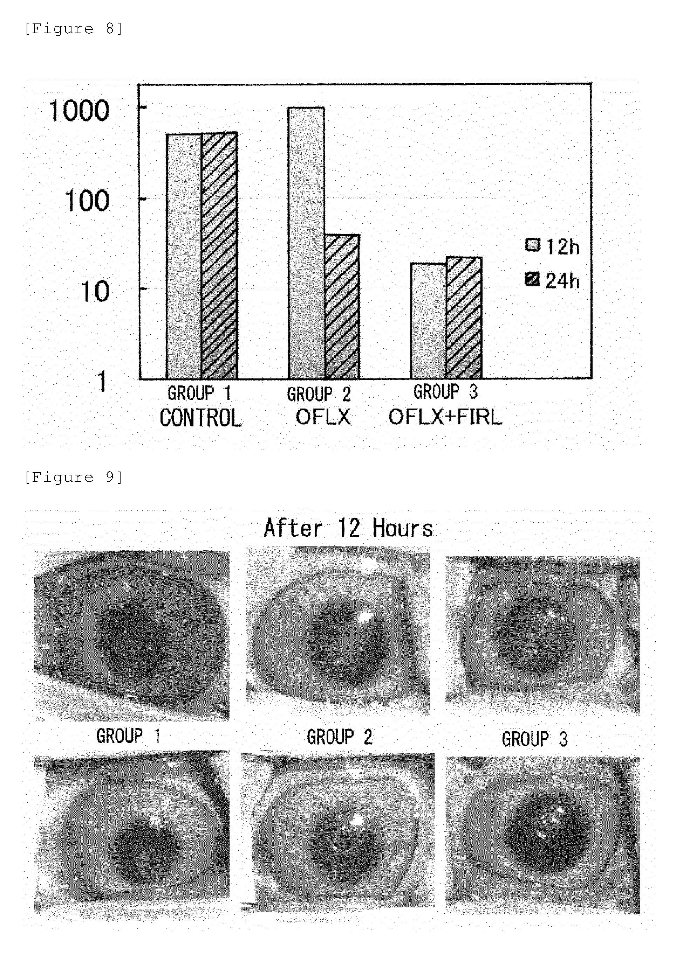

The number of bacterial cells was measured for each group 12 hours after ulcer formation (6 hours after starting the ocular instillation) and 24 hours after ulcer formation (18 hours after starting the ocular instillation). The measuring method was as follows.
Eye discharge and cornea were recovered, emulsified with a mortar after adding 5 mL of saline, and seeded on an NAC agar medium, and the number of bacterial cells was counted after 24 hours. The results are shown in Figure 8.

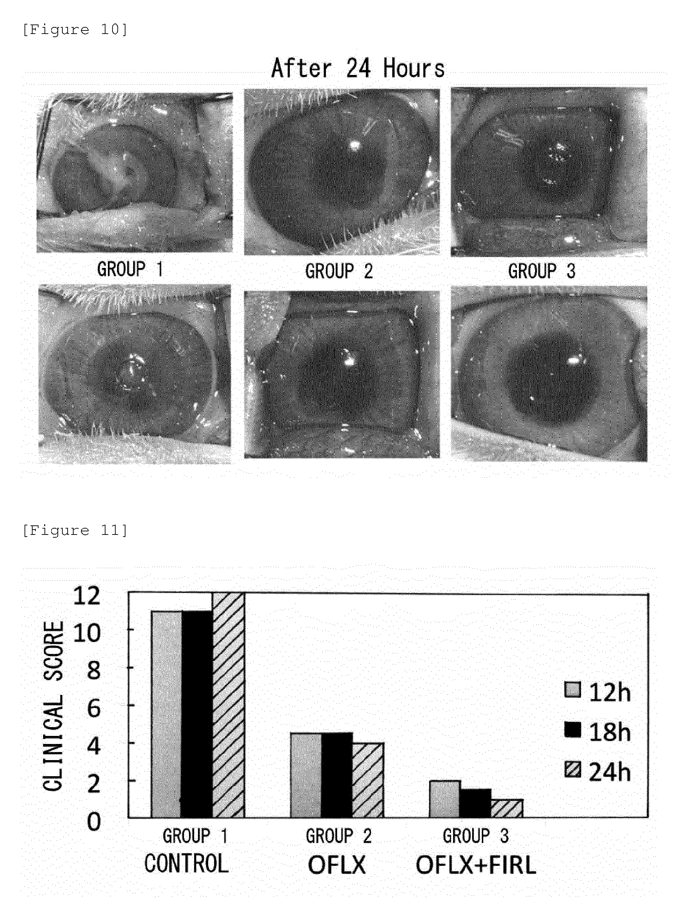

The opacity of the parenchyma of the cornea in each group was observed 12 hours after ulcer formation (6 hours after starting the ocular instillation), 18 hours after ulcer formation (12 hours after starting the ocular instillation) and 24 hours after ulcer formation (18 hours after starting the ocular instillation) to determine the clinical score by the following method.
The observed area is divided into 12 quadrants about a corneal ulcer portion, and the presence (or absence) of opacity in each quadrant is quantified in the form of "presence of opacity: 1" and "absence of opacity: 0" to calculate a clinical score (a total value of scores in 12 quadrants) for each specimen.
The appearances of the cornea in specimens of each group 12 hours and 24 hours after ulcer formation are shown in Figures 9 and 10, and changes in the clinical score 12 hours to 24 hours after ulcer formation are shown in Figure 11.

As demonstrated by the results shown in Figures 8 to 11, whereas the number of bacterial cells was increased in Group 2 to which OFLX as a conventional antibiotic was administered compared to that in the control group (Group 1) at the time of 12 hours after ulcer formation, the number of bacterial cells was dramatically decreased 12 hours after the formation in Group 3 to which the Gram-negative bacteria agent (FIRL) of the present invention was simultaneously administered; thus, the Gram-negative bacteria agent of the present invention was excellent in immediate effectivity. In the clinical score, it was also shown that the opacity was early eliminated with the effect increased in Group 3 to which the Gram-negative bacteria agent of the present invention was simultaneously administered compared to that in Group 2 to which only OFLX was administered.

### (Example 8)

### Experiment Using Mouse Pneumonia Model by Pseudomonas aeruginosa Infection

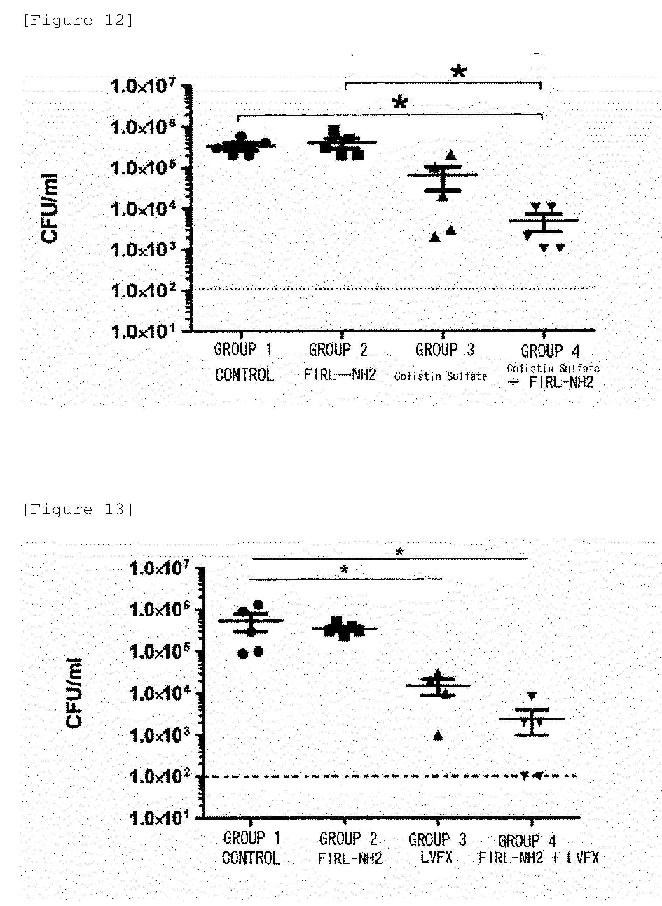

The trachea of Balb/C mice (7 to 8 weeks old, female) was exposed to 10⁷ CFU/mouse of Pseudomonas aeruginosa (strain PAO1) to make pneumonia model mice. Mice was divided into the following 4 groups (of 5 mice each), and 30 µL each of the following agents was transtracheally administered to the respective group simultaneously with Pseudomonas aeruginosa infection.
Group 1: saline (control)
Group 2: amidated FIRL (2.5 mM)
Group 3: colistin sulfate (4.7 mg/kg)
Group 4: a mixed solution of amidated FIRL (2.5 mM) and colistin sulfate (4.7 mg/kg)
Mice were sacrificed after 6 hours, and the number of bacterial cells (CFU) was measured. The results are shown in Figure 12.

Then, the number of bacterial cells (CFU) was measured in Groups 1 to 4 as before except for setting the concentration of the amidated FIRL at 5.0 mM and replacing colistin sulfate (4.7 mg/kg) with levofloxacin (LVFX) (5.0 mg/kg). The results are shown in Figure 13.

The results set forth in Figures 12 and 13 show that while no immediate effectivity against the pneumonia model was observed for the amidated FIRL alone (Group 2), the simultaneous administration thereof with each antibiotic provided a synergistic effect and decreased the number of bacterial cells about 10-fold compared to that for Group 3 to which only the antibiotic was administered.

SEQUENCE LISTING

<110> TOKAI University
<120> Anti-Gram Negative Bacteria Agents
<130> PC0490GDP
<150> JP 2009-143600
<151> 2009-6-16
<160> 6
<170> Patent In version 3.1

<210> 1
<211> 4
<212> PRT
<213> Artificial Sequence
<220>
<223> Synthesized Polypeptide
<400> 1
Leu Thr Leu Arg 4

<210> 2
<211> 6
<212> PRT
<213> Artificial Sequence
<220>
<223> Synthesized Polypeptide
<400> 2
Phe Ile Arg Leu His Pro 6
                5
<210> 3
<211> 4
<212> PRT
<213> Artificial Sequence
<220>
<223> Synthesized Polypeptide
<400> 3
Phe Ile Arg Leu 4

<210> 4
<211> 4
<212> PRT
<213> Artificial Sequence
<220>
<223> Synthesized Polypeptide
<400> 4
Ile Arg Leu His 4

<210> 5
<211> 324
<212> PRT
<213> Pseudomonas aeruginosa
<220>
<400> 5
Met Gln Val Lys His Leu Leu Leu Ile Ala Ile Leu Ala Leu Thr Ala Ala Cys Ser Ser Asn

                5                   10                  15                  20
Lys Glu Thr Val Asp Glu Asn Leu Ser Glu Ser Gln Leu Tyr Gln Gln Ala Gln Asp Asp Leu
            25                  30                  35                  40
Asn Asn Lys Scr Tyr Asn Scr Ala Val Thr Lys Lcu Lys Ala Lcu Glu Scr Arg Tyr Pro Phc
45 50 55 60
Gly Arg Tyr Ala Glu Gln Ala Gln Leu Glu Leu Ile Tyr Ala Asn Tyr Lys Asn Met Glu Pro
65 70 75 80
Glu Ala Ala Arg Ala Ala Ala Glu Arg Phe Lie Arg Leu His Pro Gln His Pro Asn Val Asp
85 90 95 100
Tyr Ala Tyr Tyr Leu Lys Gly Leu Ser Ser Phe Asp Gln Asp Arg Gly Leu Leu Ala Arg Phe
105 110 115 120
Leu Pro Lue Asp Met Thr Lys Arg Asp Pro Gly Ala Ala Arg Asp Ser Phe Asn Glu Phe Ala
125 130 135 140
Ala Gln Lcu Thr Scr Arg Phc Pro Asn Scr Arg Tyr Ala Pro Asp Ala Lys Ala Arg Mct Val
145 150 155 160
Tyr Leu Arg Asn Leu Leu Ala Ala Tyr Glu Val His Val Gly His Tyr Tyr Leu Lys Arg Gln
165 170 175 180
Ala Tyr Val Ala Ala Ala Asn Arg Gly Arg Tyr Val Val Glu Asn Phe Gln Glu Thr Pro Ala
185 190 195 200
Val Gly Asp Gly Leu Ala Ile Met Val Glu Ala Tyr Arg Arg Leu Gly Leu Asp Asp Leu Ala
205 210 215 220
Ser Thr Ser Leu Glu Thr Leu Lys Leu Asn Tyr Pro Asp Asn Ala Ser Leu Lys Asp Gly Glu
225 230 235 240
Phe Val Ala Arg Glu Ser Glu Ala Asp Thr Arg Ser Trp Leu Ala Lys Ala Thr Leu Gly Leu
245 250 255 260
Ile Glu Gly Gly Glu Pro Pro Pro His Met Glu Thr Gln Ala Ala Lys Asp Val Ile Lys Gln
265 270 275 280
Tyr Glu Asp Ala Glu Arg Glu Ile Pro Ala Glu Leu Lys Pro Glu Asn Gln Asp His Ser Ala
285 290 295 300
Asp Asp Glu Lys Pro Glu Scr Asp Asp Glu Asp Scr Gly Arg Scr Trp Trp Scr Tyr Mct Thr
305 310 315 320
Phe Gly Leu Phe 324

<210> 6
<211> 360
<212> PRT
<213> Pseudomonas aeruginosa
<220>
<400> 6
Met Val Gln Trp Lys His Ala Ala Leu Leu Ala Leu Ala Leu Ala Val Val Gly Cys Ser Asn
                5                   10                  15                  20
Ser Lys Lys Glu Leu Pro Pro Ala Glu Leu Thr Asp Phe Lys Glu Glu Val Val Leu Ser Lys
            25                  30                  35                  40
Gln Trp Ser Arg Ser Val Gly Asp Gly Gln Gly Asp Leu Tyr Asn Leu Leu Glu Pro Ala Val
        45                  50                  55                  60
Asp Gly Ser Thr Ile Tyr Ala Ala Ser Ala Glu Gly Arg Val Mat Ala Ile Gln Arg Glu Thr
65 70 75 80
Gly Asp Val Leu Trp Lys Lys Asp Leu Glu Arg Pro Val Ser Gly Gly Val Gly Val Gly Tyr
85 90 95 100
Gly Lcu Val Lcu Val Gly Thr Lcu Arg Gly Asp Val Ilc Ala Lcu Asp Glu Ala Thr Gly Lys
105 110 115 120
Lys Trp Thr Lys Arg Val Asn Ser Glu Val Leu Ser Ala Pro Ala Thr Asn Gly Asp Val Val
125 130 135 140

Val Val Gln Thr Gln Asp Asp Lys Leu Ile Gly Leu Asp Ala Ala Ser Gly Asp Gln Arg Trp
145 150 155 160
Ile Tyr Glu Ser Thr Val Pro Val Leu Thr Leu Arg Gly Thr Gly Ala Pro Leu Ile Ala Gly
165 170 175 180
Asn Met Ala Leu Ala Gly Leu Ala Ser Gly Lys Val Val Ala Val Asp Val Gln Arg Gly Leu
185 190 195 200
Pro Ile Trp Glu Gln Arg Val Ala Ile Pro Gln Gly Arg Ser Glu Leu Asp Arg Val Val Asp
205 210 215 220
Ile Asp Gly Gly Leu Leu Leu Ser Gly Asp Thr Leu Tyr Val Val Ser Tyr Gln Gly Arg Ala
225 230 235 240
Ala Ala Leu Asp Val Asn Ser Gly Arg Leu Leu Trp Aln Arg Glu Ala Ser Ser Tyr Val Gly
245 250 255 260
Val Ala Glu Gly Phe Gly Asn Ile Tyr Tyr Ser Gln Ala Ser Gly Ser Val Glu Gly Leu Asp
265 270 275 280
Ser Arg Gly Ala Ser Ser Leu Trp Asn Asn Asp Ala Leu Ala Arg Arg Gln Leu Ser Ala Pro
285 290 295 300
Ala Val Phe Ser Ser Asn Val Val Val Gly Asp Leu Glu Gly Tyr Val His Leu Leu Ser Gln
305 310 315 320
Val Asp Gly Arg Phe Val Gly Arg Glu Arg Val Asp Ser Asp Gly Val Arg Val Arg Pro leu
325 330 335 340
Val Val Gly Ser Trp Met Tyr Val Phe Gly Asn Gly Gly Lys Leu Val Ala Tyr Thr Ile Arg
345 350 355 360
360

## Claims

1. An anti-Gram-negative bacteria agent, wherein the agent exerts a bactericidal action, a growth-inhibiting action, and/or a drug efflux-inhibiting action on Gram-negative bacteria by inhibiting the formation of a YaeT complex.

2. The anti-Gram-negative bacteria agent according to claim 1, wherein the agent inhibits the binding of YaeT to TfgL.

3. The anti-Gram-negative bacteria agent according to claim 2, wherein the agent is a peptide molecule comprising an amino acid sequence consisting of at least LTLR.

4. The anti-Gram-negative bacteria agent according to claim 1, wherein the agent inhibits the binding of YaeT to YfiO.

5. The anti-Gram-negative bacteria agent according to claim 4, wherein the agent is a peptide molecule comprising an amino acid sequence consisting of at least FIRL or IRLH.

6. The anti-Gram-negative bacteria agent according to claim 5, wherein the agent is a peptide molecule comprising an amino acid sequence consisting of at least FIRLHP.

7. The anti-Gram-negative bacteria agent according to claim 3, 5 or 6, wherein the peptide molecule has an amidated C-terminal.

8. A therapeutic agent for Gram-negative bacterial infection, wherein the agent comprises the anti-Gram-negative bacteria agent according to any one of claims 1 to 7 and an antibiotic.
